# EUROPEAN PATENT APPLICATION

(11) **EP 4 681 724 A1**
(43) Date of publication of application: **21.01.2026**
(21) Application number: 24770037.0
(22) Date of filing: 15.03.2024
(51) Int. Cl.: A61K 38/17, A61P 29/00, A61P 31/04, C07K 14/73

(54) **PROTEIN FOR INHIBITING EXCESSIVE INFLAMMATORY RESPONSE AND USE THEREOF**

(30) Priority: 15.03.2023 CN 202310249618
(71) Applicant: Shanghai Institute of Material Medica, Chinese Academy of Sciences, Shanghai 201203 (CN)
(72) Inventor: TANG, Hong, Shanghai 200025 (CN); SHI, Lina, Shanghai 200025 (CN); ZHANG, Shengyuan, Shanghai 200025 (CN); XU, Qiuping, Shanghai 200025 (CN); YUAN, Feng, Shanghai 200025 (CN); LI, Fengying, Shanghai 200025 (CN); HUANG, Jie, Shanghai 200025 (CN)
(74) Representative: Nederlandsch Octrooibureau
(86) International application number: PCT/CN2024/082003
(87) International publication number: WO 2024/188347

(57) **Abstract**

Provided are a protein for inhibiting hyperinflammatory responses and a use thereof. The protein comprises a soluble CD4 (sCD4) molecule, and the use is to prevent or treat hyperinflammatory responses in sepsis or hyperinflammatory responses in macrophages. Also provided are that an effect target of the sCD4 for inhibiting the hyperinflammatory responses in macrophages is a cell membrane type MHC II, and the sCD4 plays a role by adjusting an MHC II/STING/SHP2 compound.

## Description

### FIELD OF THE DISCLOSURE

The present disclosure belongs to the field of biomedicine, and more specifically, the present disclosure relates to a recombinant protein for inhibiting hyperinflammatory responses and use in preventing or treating sepsis thereof.

### BACKGROUND OF THE DISCLOSURE

Hyperinflammation due to excessive activation of innate immune cells, leads to pathological alterations and correlates with the morbidity and mortality of infections. Innate inflammation therefore has to be tightly controlled by a series of negative regulators, at multiple levels, to maintain immunological homeostasis. Naive T cells have been demonstrated to play a crucial role in suppressing the acute innate inflammatory response to β-coronavirus and Gram-negative bacteria infections. Through direct cell-cell contact with macrophages, T cells inhibit the expression of inflammatory factors such as tumor necrosis factor (TNF) and interleukin-6 (IL-6) that are produced upon the activation of Toll-like receptors (TLRs) in macrophages. More importantly, the effective control of TLR-mediated inflammatory pathological responses in antigen-presenting cells (APCs), including macrophages, dendritic cells, and B cells, depends on the quantity rather than the quality of naive T cells. In other words, the occurrence of critical illness and/or high mortality in infected individuals with immunocompromise is directly associated with low counts of naive T cells.

In the very early stages of bacterial and viral sepsis development, T cells often undergo a sharp numerical reduction (T lymphopenia) due to massive cell death. This phenomenon is also linked to early macrophage hyperinflammatory responses and late-stage immune paralysis, which collectively contribute to multiple organ dysfunction/failure. Over the past three decades, more than 200 clinical studies targeting sepsis treatment have failed. These studies aimed to increase T cells, reverse T cell exhaustion and inhibit hyperinflammatory responses of the innate immune system (including neutralizing various inflammatory factors). This failure highlights the necessity and urgency of intensifying research into the pathogenesis of sepsis and the development of novel therapeutic strategies.

CD4 is a transmembrane protein whose ectodomain comprises four immunoglobulin (Ig)-like functional domains (D1-D4). Among these domains, the membrane-distal D1-D2 domains bind to the β₂ domain of major histocompatibility complex class II (MHC-II) molecules. Acting as a co-receptor for TCR, CD4 enhances the antigen-presenting function of MHC-II and the activation of CD4+ T cells by APCs. Additionally, CD4 serves as a receptor for the HIV envelope protein gp120, mediating the entry of HIV into CD4⁺ T cells and subsequent infection of these cells.

Upon the activation of CD4+ T cells, the ectodomain of CD4 can be cleaved by enzymatic hydrolysis to form soluble CD4 (sCD4). Early clinical studies detected sCD4 in the serum of patients with infectious diseases, autoimmune diseases and cancer. However, its physiological and pathological mechanisms have not been reported. Since the early 1990s, numerous studies have attempted to use sCD4 protein and its various derivatives to inhibit HIV infection. However, there are no reports on whether sCD4 protein has a regulatory effect on macrophage hyperinflammatory response (related) diseases.

### SUMMARY OF THE DISCLOSURE

The purpose of the present disclosure is to provide a recombinant protein sCD4 for inhibiting macrophage hyperinflammatory responses and a use thereof in the manufacture of a pharmaceutical composition for preventing or treating hyperinflammatory responses in sepsis.

In the first aspect, the present disclosure provides a use of a recombinant soluble CD4 protein or a construct expressing the recombinant soluble CD4 protein (for example, an expression vector; more specifically, an adenoviral vector, a lentiviral vector, etc.) in the manufacture of a pharmaceutical composition for preventing or treating inflammatory responses, wherein the inflammatory responses are septic hyperinflammation or inflammatory responses in macrophages.

In one or more preferable embodiments, the inflammatory responses in macrophages comprise an inflammation caused by elevated expression of inflammatory factors mediated by TLR4 in macrophages in the onset of sepsis.

In one or more preferable embodiments, the inflammation caused by elevated expression of inflammatory factors mediated by TLR4 in macrophages comprises: an inflammation caused by elevated TNF expression and/or an inflammation caused by elevated IL-6 expression.

In one or more preferable embodiments, the recombinant soluble CD4 protein comprises the ectodomain of the CD4 molecule.

In one or more preferable embodiments, the ectodomain of the CD4 molecule comprises D1, D2, D3 and D4 domains.

In one or more preferable embodiments, the recombinant soluble CD4 protein is encoded by a gene codon-optimized coding sequence of human or mouse origin.

In one or more preferable embodiments, the recombinant soluble CD4 protein comprises a protein with the amino acid sequence shown in SEQ ID NO: 1 or SEQ ID NO: 3 or a conservative variant thereof (a variant with the sequence and the function of the protein as shown in SEQ ID NO:1 or SEQ ID NO:3).

In one or more preferable embodiments, the conservative variant is selected from: (1) a derivative protein formed by substituting, deleting or adding one or several (such as 1-20; preferably 1-10; more preferably 1-5, 1-3 or 1-2) amino acid residues in the amino acid sequence shown in SEQ ID NO: 1 or SEQ ID NO: 3 and retaining the function of the amino acid sequence shown in SEQ ID NO: 1 or SEQ ID NO: 3; (2) a derivative protein with an amino acid sequence having more than 80% identity (preferably, more than 85%, 90%, 95% identity, such as more than 98% or 99% identity) to the amino acid sequence shown in SEQ ID NO: 1 or SEQ ID NO: 3 and retaining the function of the amino acid sequence shown in SEQ ID NO: 1 or SEQ ID NO: 3; or (3) a protein with a tag or a signal polypeptide fused to the protein (such as at the N-terminus or C-terminus) with the amino acid sequence shown in SEQ ID NO: 1 or SEQ ID NO: 3.

In one or more preferable embodiments, the conservative variant is selected from (but is not limited to): a variant comprising a small peptide tag that facilitates the detection of the protein with the amino acid sequence shown in SEQ ID NO: 1 or SEQ ID NO: 3, and/or an Fc-fused recombinant variant that enhances the stability of the protein with the amino acid sequence shown in SEQ ID NO: 1 or SEQ ID NO: 3. In one or more preferable embodiments, the construct expressing soluble CD4 comprises (but is not limited to) an expression vector. In one or more preferable embodiments, the expression vector comprises a viral expression vector and a non-viral expression vector. In one or more preferable embodiments, the viral expression vector comprises (but is not limited to): an adeno-associated viral vector, a lentiviral vector or an adenoviral vector.

In one or more preferable embodiments, the soluble CD4 inhibits the inflammatory responses through crosstalk by binding to MHC II on macrophages; preferably, the inflammatory responses comprise septic hyperinflammation or inflammatory responses in macrophages; more preferably, the inflammatory responses in macrophages are TLR4-mediated inflammatory responses in macrophages.

In one or more preferable embodiments, the TLR4-mediated inflammatory responses are mediated by a signaling pathway of MHC II-TLR4 inflammatory response complex in macrophages; preferably, the signaling pathway comprises an up-regulation of MHC II by the recombinant soluble CD4 protein or the construct expressing soluble CD4, a recruitment and activation of SHP-2, an inhibition of TRAF6 and an inhibition of TLR4/NF-κB-mediated inflammatory response through crosstalk.

In one or more preferable embodiments, the recombinant soluble CD4 protein or the construct expressing soluble CD4 may be comprised in a pharmaceutically acceptable carrier to form a pharmaceutical composition.

In another aspect, the present disclosure provides a use of an artificially established or intracellular signaling pathway or a system comprising the signaling pathway for screening substances (comprising compounds, compositions, drugs, etc.) that inhibit inflammatory responses in macrophages; wherein the signaling pathway is selected from the group consisting of: MHC II-SHP2 and/or STING-TRAF6-TLR4 in macrophages; MHC II-TLR4 in macrophages; SHP2-TRAF6-TLR4 in macrophages; STING- TLR4 in macrophages.

In another aspect, the present disclosure provides a method for screening substances that inhibit inflammatory responses in macrophages, comprising:
(1) contacting a candidate substance with a system comprising a signaling pathway selected from the group consisting of: MHC II-SHP2 and/or STING-TRAF6-TLR4 in macrophages; MHC II-TLR4 in macrophages; SHP2-TRAF6-TLR4 in macrophages; STING- TLR4 in macrophages;
(2) observing the system in (1) and screening out substances that have a regulation on the signaling pathway in (1), wherein the substances (comprising potential substances) are useful for inhibiting inflammatory responses in macrophages; wherein, the regulation comprises up-regulation (such as activating or increasing expression) of MHC II, up-regulation of SHP2, up-regulation of STING, and down-regulation (such as inhibiting or decreasing expression) of TRAF6.

In one or more embodiments, the signaling pathway in (1) is MHC II-SHP2-TRAF6-TLR4 in macrophages; the regulation in (2) comprises an up-regulation of MHC II, an up-regulation of SHP2, wherein the SHP2 down-regulates TRAF6, thereby inhibiting the TLR4-mediated inflammatory response.

In one or more embodiments, the signaling pathway in (1) is MHC II-TLR4 in macrophages; the regulation in (2) comprises an up-regulation of MHC II, thereby inhibiting the TLR4-mediated inflammatory response.

In one or more embodiments, the signaling pathway in (1) is SHP2-TRAF6-TLR4; the regulation in (2) comprises an up-regulation of SHP2, wherein the SHP2 down-regulates TRAF6, thereby inhibiting the TLR4-mediated inflammatory response.

In one or more embodiments, the signaling pathway in (1) is STING-TLR4; the regulation in (2) comprises an up-regulation of STING, thereby inhibiting the TLR4-mediated inflammatory response.

In one or more embodiments, a control group is also included in the method, wherein the control group is a system that does not comprise the candidate substance and comprises a signaling pathway selected from the group consisting of: MHC II-SHP2 and/or STING-TRAF6-TLR4 in macrophages; MHC II-TLR4 in macrophages; SHP2-TRAF6-TLR4 in macrophages; STING-TLR4 in macrophages.

In one or more embodiments, the system further comprises MyD88/IRAK1/TRAF6 inflammatory signaling molecules downstream of TLR4; thereby, the method further comprises: observing the activation status of the MyD88/IRAK1/TRAF6 inflammatory signaling molecules downstream of TLR4; if their activation is inhibited, it indicates that the candidate substance is a substance useful for inhibiting inflammatory responses in macrophages.

In one or more embodiments, the system further comprises NF-κB, wherein the NF-κB drives the expression of inflammatory cytokine genes such as TNF/IL-6; thereby, the method further comprises: observing the expression of inflammatory cytokines (such as TNF/IL-6, etc.) driven by NF-κB; if the expression is decreased, it indicates that the candidate substance is a substance useful for inhibiting inflammatory responses in macrophages.

In one or more embodiments, the inhibition status of the TLR4 inflammatory response is evaluated based on the expression or presence of inflammatory cytokines; preferably, the inflammatory cytokines comprise: TNF and/or IL-6; if the expression or presence of TNF and/or IL-6 shows no significant change (for example, no significant downregulation compared to the control group), then the TLR4 inflammatory response is not inhibited; if the expression of TNF and/or IL-6 is significantly decreased, then the TLR4 inflammatory response is inhibited.

In one or more embodiments, the system comprising the signaling pathway is selected from: a cellular (culture) system, a subcellular (culture) system, a tissue (culture) system or an animal system.

In one or more embodiments, the candidate substance comprises but is not limited to: regulatory molecules (such as, but not limited to, up-regulators, interfering molecules, nucleic acid inhibitors, binding molecules (such as antibodies or ligands)) designed for the signaling pathway or the proteins thereof, or upstream or downstream proteins or genes thereof; CRISPR constructs; small molecule compounds; compounds from compound libraries.

In one or more embodiments, the increase or up-regulation is a statistically significant increase or up-regulation, such as an increase or up-regulation by at least 10% or 20% compared to the control or baseline, preferably an increase or up-regulation by at least 40% or 50%, more preferably an increase or up-regulation by at least 80% or 100%.

In one or more embodiments, the inhibition is a form of downregulation and is a statistically significant inhibition or downregulation, such as an inhibition or downregulation by at least 10% or 20% compared to the control or baseline, preferably an inhibition or downregulation by at least 40% or 50%, more preferably an inhibition or downregulation by at least 80% or 100%.

Other aspects of the present disclosure will be apparent to those skilled in the art based on the disclosure herein.

### DESCRIPTION OF FIGURES

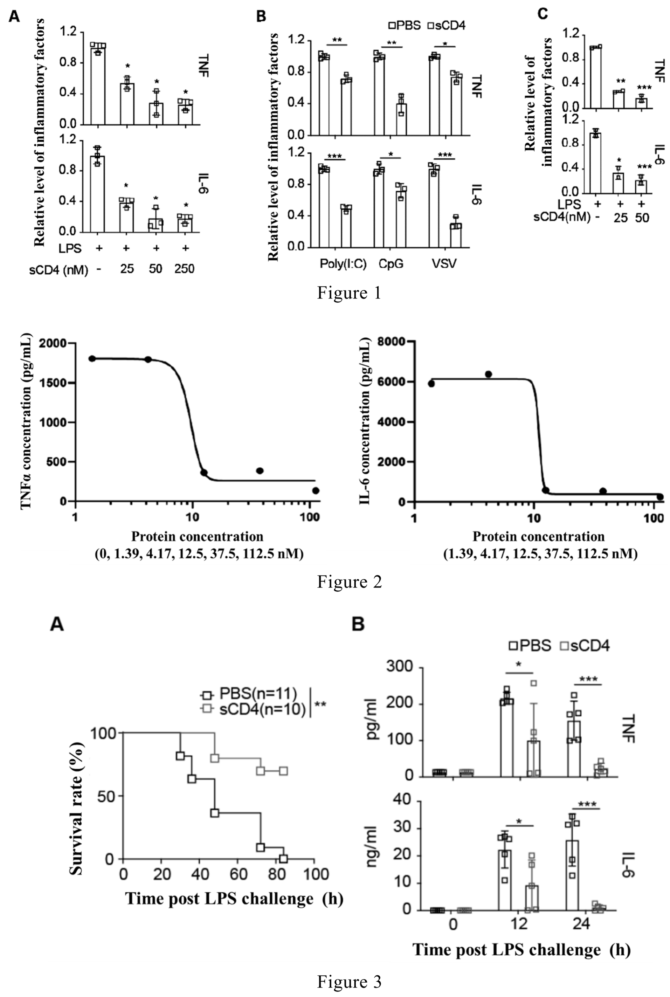

Figure 1. sCD4 inhibits TLR-mediated inflammatory responses in APCs (macrophages, dendritic cells). (A) Mouse bone marrow-derived macrophages (BMDM) were co-cultured with different concentrations of murine sCD4 protein (25 nM, 50 nM, 250 nM). The concentrations of inflammatory cytokines TNF and IL-6 in the cell supernatant were measured by Luminex assay. (B) After co-culture of BMDM with recombinant murine sCD4 protein, the TLR3, TLR7, and TLR9 signaling pathways were activated using poly I:C, CpG and VSV virus, respectively. (C) Mouse bone marrow-derived dendritic cells (BMDC) were co-cultured with different concentrations of murine sCD4 protein (25 nM, 50 nM). The inhibitory effect of sCD4 on inflammatory responses triggered by different TLRs or in different cells was detected using the same method described above.
Figure 2. Human sCD4 inhibits TLR4 inflammatory response in macrophages. Human peripheral blood mononuclear cell (PBMC)-derived macrophages were co-cultured with different concentrations of human sCD4 protein (1.39 nM, 4.17 nM, 12.5 nM, 37.5 nM, 112.5 nM), then challenged with LPS for the indicated duration. The concentrations of inflammatory cytokines in the supernatant were measured by Luminex assay.
Figure 3. sCD4 protein effectively reduces mortality in LPS-induced sepsis. (A) Mice were intraperitoneally administered sCD4 protein followed by intraperitoneal injection of LPS, and mouse survival rate was monitored. (B) The concentrations of TNF and IL-6 in mouse serum were detected by Luminex assay.

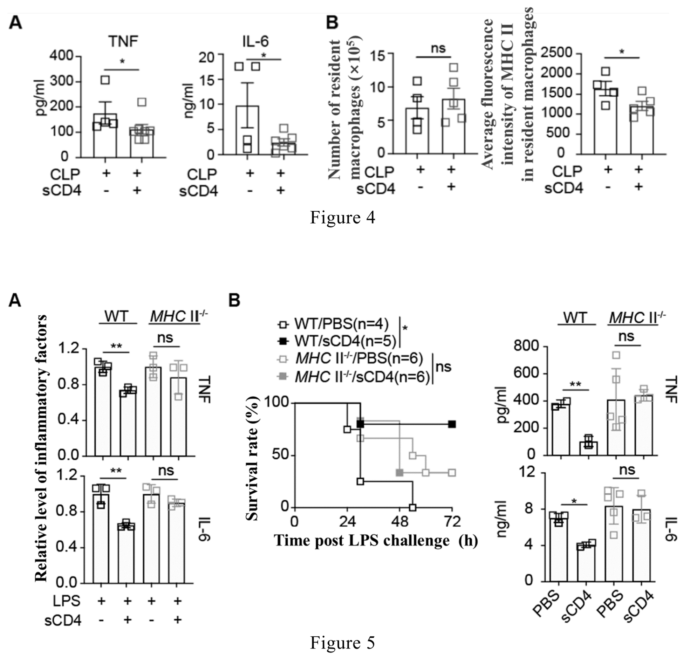

Figure 4. sCD4 protein effectively reduces mortality in CLP-induced sepsis. (A) Mice were intraperitoneally administered sCD4 protein followed by cecal ligation and puncture (CLP) surgery. (A) The inhibitory effect of sCD4 on mouse inflammatory cytokine response was detected by Luminex assay. (B) Flow cytometric analysis of sCD4-mediated inhibition of splenic macrophage activation.

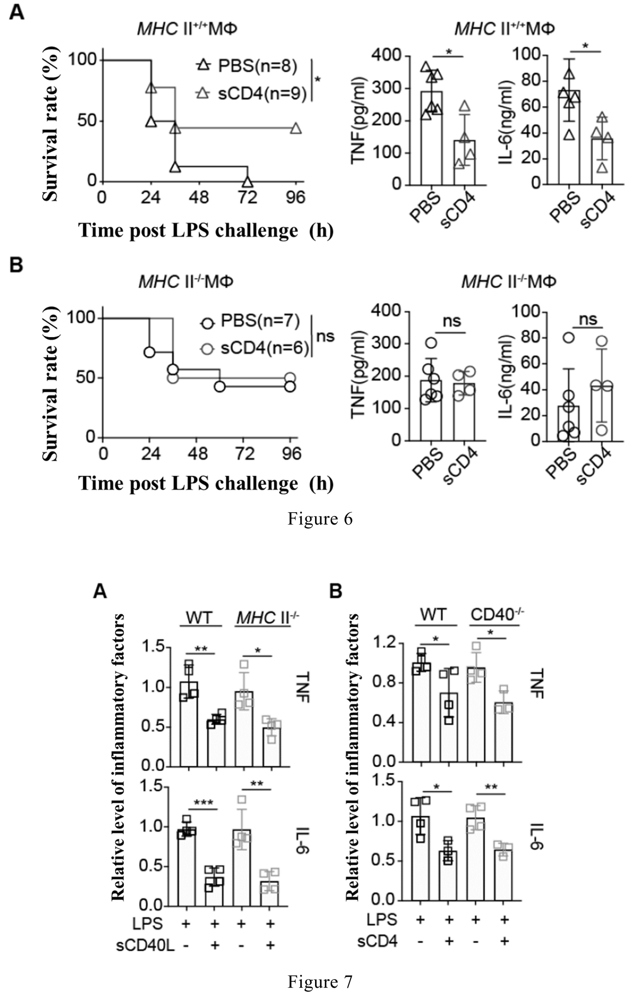

Figure 5. sCD4 inhibition of TLR4 inflammatory response depends on MHC II. (A) sCD4 was co-cultured with BMDM from either wild-type (WT) or MHC II knockout (MHC II^{-/-}) mice, followed by LPS challenge; sCD4 failed to prevent the TLR4 inflammatory response in MHC II^{-/-} BMDM. (B) sCD4 protein was intraperitoneally injected into WT or MHC II^{-/-} mice, followed by LPS challenge; sCD4 protein failed to reduce mortality or the cytokine storm in MHC II^{-/-} mice. Figure 6. sCD4 inhibition of TLR4 inflammatory response depends on MHC II molecules in macrophages. After depleting macrophages in mice using clodronate liposomes, MHC II^{+/+} (A) or MHC II^{-/-}(B) macrophages were adoptively transferred. Mice were then intraperitoneally injected with sCD4 protein, followed by intraperitoneal LPS injection. Survival rate of mice and serum inflammatory cytokine levels were subsequently monitored.
Figure 7. sCD4 inhibition of macrophage TLR4 inflammatory response is independent of the CD40 anti-inflammatory signaling pathway. (A) sCD40 ligand protein (sCD40L) was co-incubated with either wt or MHC II^{-/-} BMDM. (B) sCD4 was co-incubated with either wt or CD40 knockout (CD40^{-/-}) BMDM. After LPS challenge, the levels of inflammatory cytokines in the supernatant were detected by Luminex assay.

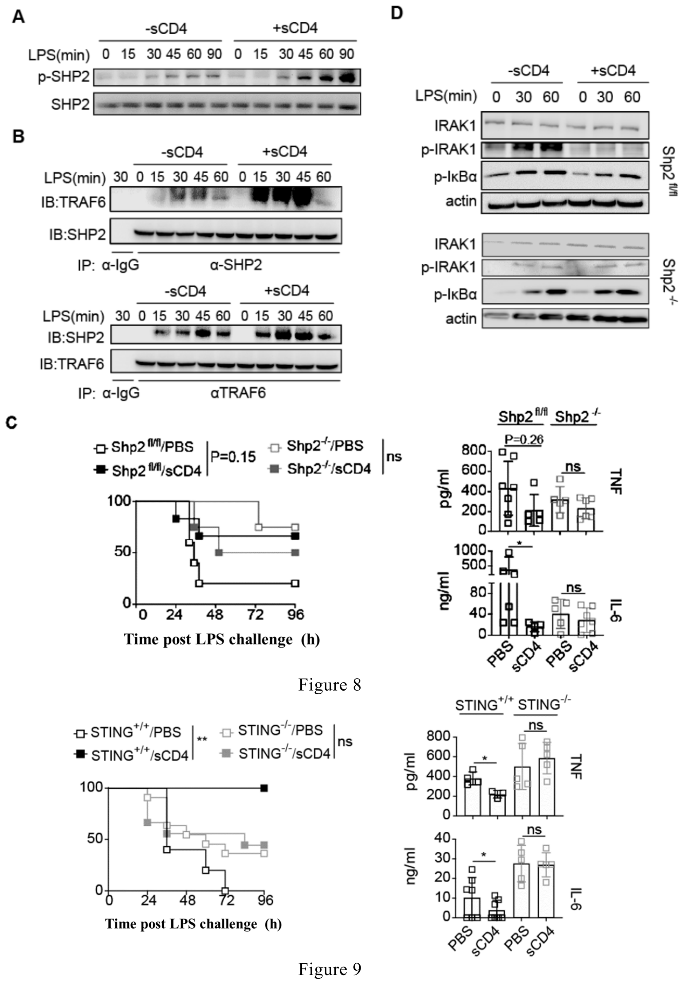

Figure 8. sCD4 modulates the TLR4 inflammatory response by activating SHP2 to inhibit TRAF6. BMDM were co-incubated with sCD4 protein, followed by LPS challenge for different durations. (A) Phosphorylation levels of SHP2 in cell lysates were detected by Western blotting. (B) Co-immunoprecipitation (co-IP) of SHP2 and TRAF6 was performed to detect their interaction. (C) Wild-type (SHP2^{fl/fl}) or macrophage-specific SHP2 knockout (SHP2^{-/-}) mice were intraperitoneally injected with sCD4 protein, followed by intraperitoneal LPS injection; survival rate of mice and inflammatory response capacity were detected. (D) Changes in the TLR4 inflammatory response, including detection of IRAK1 and IκBα phosphorylation levels, in wild-type (SHP2^{fl/fl}) or SHP2^{-/-} BMDM pre-treated with sCD4 protein.
Figure 9. sCD4 inhibition of TLR4 inflammatory response also depends on STING. sCD4 protein was intraperitoneally injected into WT (STING^{+/+}) or STING knockout (STING^{-/-}) mice, followed by intraperitoneal LPS injection. Survival rate of mice and inflammatory response capacity were detected.

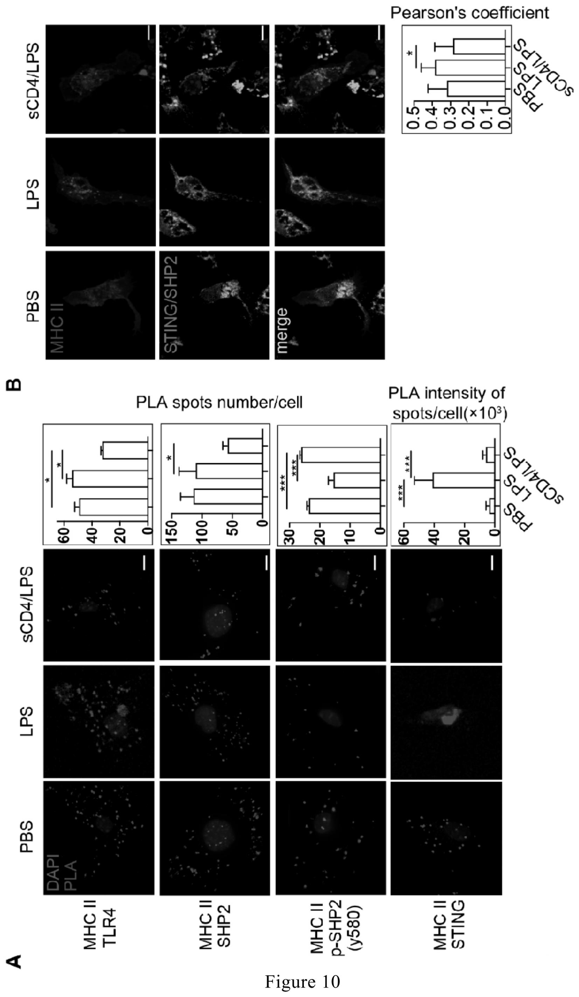

Figure 10. sCD4 disrupts the MHCII/TLR4 complex lipid raft domain and inhibits pro-inflammatory TLR4 plasma membrane localization. BMDM were pre-incubated with sCD4 followed by LPS addition. (A) Duolink assay was used to quantitatively analyze pairwise interactions (red dots) between the indicated protein molecules in BMDM. (B) Colocalization (yellow) in BMDM of Duolink-stained STING and SHP2 heterodimers (green) with immunofluorescently stained MHC II molecules (red). Nuclei were counterstained with DAPI. Pearson's coefficient indicates the degree of colocalization. Scale bar= 5 µm.

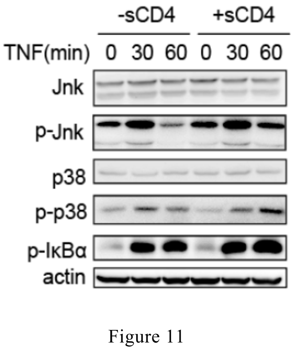

Figure 11. sCD4 specifically interferes with TLR4 activation without affecting TNF inflammatory signaling. BMDM were co-incubated with sCD4 protein, followed by TNF stimulation for different durations. Changes in the inflammatory response in cell lysates, including phosphorylation levels of JNK, p38, and IκBα, were detected by Western blotting.

### DETAILED DESCRIPTION

Through extensive research, the present inventors have revealed that soluble CD4 (sCD4) inhibits macrophage hyperinflammatory responses; simultaneously, they have uncovered the target of sCD4 inhibiting excessive inflammatory responses in macrophages, MHC II, and its crosstalk mechanism that inhibits the TLR/NF-κB inflammatory signaling cascade.

### sCD4

sCD4 is derived from the extracellular domain of the CD4 molecule. The amino acid sequence of human sCD4 can be as shown in SEQ ID NO: 3, and its nucleotide sequence can be as shown in SEQ ID NO: 4; the amino acid sequence of murine sCD4 can be as shown in SEQ ID NO: 1, and its nucleotide sequence can be as shown in SEQ ID NO: 2. The present disclosure also comprises sCD4 homologs from other species and the use thereof.

The sCD4 of the present disclosure may be naturally occurring, for example, it may be isolated or purified from mammals. In addition, the sCD4 can also be artificially prepared, for example, recombinant sCD4 can be produced according to conventional genetic engineering recombination for experimental or clinical applications. Recombinant sCD4 may be used in applications. The sCD4 comprises full-length sCD4 or a biologically active fragment thereof. Preferably, the amino acid sequence of the sCD4 can be substantially identical to the sequence shown in SEQ ID NO: 3 or SEQ ID NO: 1. The corresponding nucleotide coding sequence can be readily deduced based on the amino acid sequence of sCD4.

The amino acid sequence of sCD4 formed with substitution, deletion or addition of one or several amino acid residues is also included in the present disclosure. sCD4 or a biologically active fragment thereof comprises a sequence with substitution of several conservative amino acids, wherein the sequence with amino acid substitution does not affect its activity or retains part of its activity. Appropriate substitution of amino acids is a well-known method in the art. This method can be readily performed and ensures that the biological activity of the resulting molecule is not altered. These methods allow those skilled in the art to recognize that, in general, changes to single amino acids in non-essential regions of a polypeptide do not substantially alter its biological activity.

Any one of biologically active fragments of the sCD4 can all be applied to the present disclosure. Here, the biologically active fragment of sCD4 refers to a polypeptide that can still maintain all or part of the functions of the full-length sCD4. Usually, the biologically active fragments retain at least 50% of the activity of the full-length sCD4. Under more preferable conditions, the active fragment can maintain 60%, 70%, 80%, 90%, 95%, 99%, or 100% of the activity of the full-length sCD4.

Modified or improved sCD4 can also be used in the present disclosure, e.g., sCD4 that have been modified or improved to enhance their half-life, effectiveness, metabolism, and/or protein potency can be used. The modified or improved sCD4 may be a conjugate of sCD4, or it may comprise substituted or artificial amino acids. The modified or improved sCD4 may share little similarity with naturally occurring sCD4 but can still inhibit macrophage inflammatory responses (e.g., treating sepsis). That is, any variant that does not affect the biological activity of sCD4 can be used in the present disclosure.

The present disclosure provides a method of using a soluble CD4 protein (sCD4) capable of inhibiting excessive inflammatory responses. sCD4, by recognizing and acting on the MHC II receptor on the macrophage surface, inhibits the excessive expression of inflammatory cytokine genes mediated by macrophage TLR4, and demonstrates excellent anti-inflammatory functions in sepsis induced by Gram-negative bacterial lipopolysaccharide (LPS) and Cecal Ligation and Puncture (CLP).

In the research work of the present inventors, technical solutions including but not limited to the following aspects were elucidated for the first time:
(1) Murine and/or human sCD4 protein can inhibit the production and secretion of inflammatory cytokines TNF and IL-6 in bone marrow-derived macrophages (BMDM) and peripheral blood mononuclear cell (PBMC)-derived macrophages, respectively, in a concentration-dependent manner. sCD4 can also inhibit inflammatory responses in macrophages triggered by TLR3, TLR7 and TLR9 agonists.
(2) sCD4 can inhibit the progression of inflammation in animal models of LPS-induced and CLP-induced sepsis.
(3) sCD4 inhibits TLR4-mediated inflammatory responses in macrophages independently of CD4 T cell co-inhibitory molecules (e.g., CD40 ligand, CD40L).
(4) sCD4 specifically recognizes and utilizes MHC II to exert a negative regulatory effect on macrophage inflammatory responses and MHC II on macrophages is sufficient to mediate the control of excessive inflammatory responses like sepsis by sCD4.
(5) sCD4 specifically modulates the TLR4 inflammatory response in an MHC II-dependent manner; it does not affect other inflammatory cytokine cascades (e.g., TNFR).
(6) sCD4 binds to MHC II and the intracellular domain of the latter recruits and activates SHP2 and STING, thereby inhibiting the activation of inflammatory signaling molecules downstream of TLR4, namely MyD88/IRAK1/TRAF6, leading to the blockade of NF-κB-driven gene expression of inflammatory cytokines such as TNF/IL-6.

In specific embodiments of the present disclosure, human and murine sCD4 expressed efficiently in human HEK293 cells were obtained, and their effective drug concentrations for inhibiting TLR inflammatory responses and sepsis occurrence were determined in cultured cells and various sepsis animal models, demonstrating significant therapeutic and clinical value for CD4 T lymphopenia syndrome accompanied by inflammatory responses.

Based on the new discoveries of the present inventors, the present disclosure provides the application of sCD4 and up-regulators thereof (comprising expression constructs thereof) for preparing recombinant proteins that inhibit macrophage inflammatory responses.

As used herein, the terms "macrophage inflammatory response/symptom (macrophage inflammation)" and "macrophage hyperinflammatory responses/symptoms (macrophage hyperinflammation)" are used interchangeably and both represent clinical indications/diseases caused by macrophage hyperinflammatory responses (e.g., manifested as increased levels of TLR4-mediated inflammatory cytokines; more specifically, such as increased TNF or IL-6 levels).

As used herein, the term "septic hyperinflammatory responses/symptoms" represent clinical indications/diseases caused by septic hyperinflammatory responses.

As used herein, the up-regulator of sCD4 comprises activators, agonists, and so on. Any substance that can increase the activity of sCD4, increase the stability of sCD4, up-regulate the expression of sCD4, promote the secretion of sCD4, increase the effective time of sCD4, or promote the transcription and translation of sCD4 can be used in the present disclosure as effective substances with upregulating functions.

As a preferable embodiment of the present disclosure, the up-regulator of sCD4 comprises (but is not limited to): an expression vector or an expression construct capable of expressing (preferably over-expressing) sCD4 after being transferred into cells. Usually, the expression vector comprises a gene cDNA subcloning cassette, and the gene cassette comprises the gene encoding sCD4 and the regulatory sequences operatively linked thereto. The term "operably linked" or "operably linked to" refers to a condition that certain parts of a linear DNA sequence can regulate or control the activity of other parts of the same linear DNA sequence. For example, a promoter is operably linked to a coding sequence if it controls the transcription of the sequence.

In the present disclosure, the sCD4 polynucleotide sequence can be inserted into a recombinant expression vector, so that it can be transferred into cells and over-expressed to produce sCD4. Any plasmid and vector can be used as long as it can replicate and be stable in the hosts. An important characterastic of an expression vector is that it usually contains an origin of replication, a promoter, a marker gene, and translation control elements. For example, the expression vectors comprise: viral vectors, non-viral vectors; preferably, the expression vectors comprise (but are not limited to): adeno-associated virus, lentiviral vectors, adenoviral vectors and so on.

Those skilled in the art are well known of methods for constructing expression vectors containing DNA sequences comprising sCD4 and suitable transcription/translation control signals. These methods include in vitro recombinant DNA techniques, DNA synthesis techniques, in vivo recombination techniques, etc.

The present disclosure also provides a pharmaceutical composition, wherein it comprises effective amounts (e.g., 0.000001-20wt%; preferably 0.00001-10wt%) of the sCD4, or an up-regulator thereof, or an analogue thereof, and a pharmaceutically acceptable carrier.

The composition of the present disclosure can be directly used for inhibiting macrophage inflammatory responses. In addition, it can also be used in combination with other active agents or adjuvants at the same time.

Generally, these materials can be formulated in a non-toxic, inert medium with pharmaceutically acceptable aqueous carrier, wherein the pH is usually about 5-8, preferably the pH is about 6-8.

As used herein, the term "comprise" means that various components can be used together in the mixture or composition of the present disclosure. Therefore, the term "mainly consist of......" and "consist of......" is included in the term "comprise". As used herein, the term "effective amount" refers to an amount that is functional or active for humans and/or animals and is acceptable for administration to humans and/or animals.

As used herein, "pharmaceutically acceptable" ingredients refers to substances suitable for use in humans and/or mammals without undue adverse side effects (such as toxicity), i.e. with reasonable benefit/risk ratio. The term "pharmaceutically acceptable carrier" refers to a carrier for the administration of a therapeutic agent, comprising various excipients and diluents.

The composition of the present disclosure comprises a safe and effective amount of sCD4, or an up-regulator thereof (such as an expression vector that overexpresses the sCD4), or an analog thereof, and a pharmaceutically acceptable carrier. Such carriers comprise (but are not limited to): saline, buffer, glucose, water, glycerol, ethanol, and combinations thereof. Usually, the pharmaceutical formulation should match the route of administration. The pharmaceutical composition of the present disclosure can be prepared in the form of an injection, for example, using a saline solution or a water solution containing glucose and other excipients through conventional methods. The pharmaceutical composition should be manufactured under sterile conditions. The amount of active ingredient administered is a therapeutically effective amount. The pharmaceutical formulations of the present disclosure can also be prepared into sustained-release formulations.

The effective amount of sCD4, or an agonist thereof in the present disclosure may vary with the route of administration, the severity of the disease to be treated, and the like. Selection of preferred effective amount can be determined by those skilled in the art based on various factors (e.g. through clinical trials). Such factors comprise but are not limited to: pharmacokinetic parameters (such as bioavailability, metabolism, half-life, and so on) of the sCD4 or agonist thereof, the severity of the disease to be treated, weight, immune status of patients, the route of administration, and so on. Usually, satisfactory effects can be obtained when the sCD4 or the up-regulator of the present disclosure is administered daily at a dose of about 0.00001-10 mg/kg of animal body weight. For example, depending on the urgency of the treatment condition, several divided doses may be administered daily, or the dose may be proportionally reduced.

The present disclosure also provides a method for inhibiting macrophage inflammatory responses, comprising administering to a subject an effective amount of sCD4, or an up-regulator thereof (e.g., an expression vector overexpressing the sCD4), or an analog thereof.

The route of administrating sCD4 or the agonist of the present disclosure is not particularly limited. It may be systemic or local. For example, the sCD4 or the up-regulator of the present disclosure can be administered to animals via intraperitoneal injection, intravenous injection, oral administration, subcutaneous injection, intrathecal injection, intradermal injection, etc.

After knowing uses of the sCD4, various methods well known in the art can be used to administer the sCD4 or an coding gene thereof, or a pharmaceutical composition thereof. Preferably, gene therapy means can be used, for example, sCD4 or an up-regulator thereof can be directly administered to the subject through methods such as injection. Alternatively, expression unit carrying the sCD4 gene (such as an expression vector or virus, etc.) can be delivered to the target, allowing active expression of active sCD4.

As an embodiment of the present disclosure, the sCD4 can be directly administered to mammals (such as humans), or the gene encoding sCD4 can be cloned into an appropriate vector (such as conventional prokaryotic or eukaryotic expression vectors, or virus vectors such as herpes virus vectors or adenovirus vectors) by conventional methods, then the vectors can be introduced into cells that can express the sCD4, so that the cells express sCD4. sCD4 expression can be achieved by introducing an appropriate amount of the cells into an appropriate part in a mammal.

The mode of administration for the up-regulator or analog of sCD4 depends primarily on the type and characteristics of said up-regulator, which can be evaluated by those skilled in the art.

Although specific examples of the present disclosure provide dosing regimens for animals such as mice, it should be understood that converting the dosage from animals such as mice to a dosage suitable for humans is readily achievable by those skilled in the art, for example, it can be calculated according to the Meeh-Rubner formula: Meeh-Rubner formula: A=k×(W^{2/3})/10,000. In the formula, A is the body surface area, calculated in m²; W is the body weight, calculated in g; K is a constant, which varies with animal species. Generally speaking, mice and rats are 9.1, guinea pigs are 9.8, rabbits are 10.1, cats are 9.9, dogs are 11.2, monkeys are 11.8, human is 10.6. It will be understood that, depending on the drug and the clinical situation, the conversion of the administered dose may vary according to the assessment of an experienced pharmacist.

The present disclosure can provide a new paradigm for the prevention or treatment of sepsis. Applying sCD4 protein to target inflammatory cells, its "root-cause addressing" mechanism and potency are significantly superior to current "symptomatic" treatment regimens such as corticosteroid therapy or antibodies targeting inflammatory cytokines (TNF, IL-6, etc.) or their cognate receptors, or, through combined administration, can significantly reduce the drug concentration and/or frequency of use of corticosteroids or inflammatory cytokine antibodies.

### Macrophage-Related Signaling Pathways and The Regulation thereof

As used in the present disclosure, the term "(signaling) pathway" refers to a signaling system formed by a series of genes, proteins, or metabolic products (synthetic or processed products) that interact or mutually regulate each other, also comprises the interactions between pathway proteins and other cellular components or organelles, and sometimes involves the participation of their upstream or downstream genes or proteins, wherein these upstream or downstream genes or proteins generally lead to the occurrence of some cellular events. The macrophage-related signaling pathway primarily comprises the following key elements: macrophage MHC II, TRAF6, Toll-like receptor (TLR), SHP2 or STING. Among these, the macrophage-related signaling pathway comprises the involvement of autophagosomes and lysosomes.

As used in the present disclosure, the terms "(signaling) pathway" and "(signaling) route" can be used interchangeably.

The protein amino acid sequence of the MHC II is, for example, as shown in GenBank: NP_996988.2 (murine MHC II beta chain); NP_034508.2 (murine MHC II A/Eα chain). The corresponding nucleotide sequences are A/Eα (NM_010378.3) and Aβ (NM_207105.3), respectively.

The protein amino acid sequence of the SHP2 is, for example, as shown in GenBank Q06124 (murine).

The protein amino acid sequence of the STING is, for example, as shown in GenBank Q86WV6 (murine).

The protein amino acid sequence of the TRAF6 is, for example, as shown in GenBank_Q9Y4K3 (murine).

The protein amino acid sequence of the TLR4 is, for example, as shown in GenBank_O00206 (murine).

In the present disclosure, unless otherwise stated, the signaling pathway downstream of TLR4 leading to inflammation is known to those skilled in the art, and information regarding upstream and downstream proteins/genes is also known to those skilled in the art.

In the present disclosure, the pathway can comprise a pathway selected from the following group: MHC II-SHP2 and/or STING-TRAF6-TLR4 in macrophages; MHC II-TLR4 in macrophages; SHP2-TRAF6-TLR4 in macrophages; STING-TLR4 in macrophages. The sCD4 inhibits the occurrence of macrophage inflammatory responses (inflammation) by regulating the pathway. The upstream, intermediate or downstream parts of the pathway may also comprise other regulatory genes or upstream/downstream pathways.

It should be understood that, upon knowing the function of the macrophage-related signaling pathway (preferably, comprising upstream and downstream proteins or genes thereof), various methods well-known to those skilled in the art can be employed to regulate said macrophage-related signaling pathway. For example, by up-regulating the expression level or activation level of pathway proteins, the goal of inhibiting inflammation can be achieved.

As a preferable embodiment of the present disclosure, based on the MHC II-SHP2 and/or STING-TRAF6-TLR4 signaling pathway in macrophages, a substance that activates/up-regulates macrophage MHC II (e.g., sCD4, or a construct overexpressing MHC II) is provided. By activating MHC II, its intracellular domain recruits and activates SHP2 and STING, thereby inhibiting the activation of the inflammatory signaling molecules MyD88/IRAK1/TRAF6 downstream of TLR4, leading to the blockade of NF-κB-driven gene expression of inflammatory cytokines such as TNF/IL-6.

As a preferable embodiment of the present disclosure, based on the MHC II-TLR4 pathway in macrophages, a substance that activates/up-regulates macrophage MHC II (e.g., sCD4, or a construct overexpressing MHC II) is provided. It inhibits the TLR4 inflammatory response by activating MHC II.

As a preferable embodiment of the present disclosure, based on the SHP-TRAF6-TLR4 pathway in macrophages, a substance that activates/up-regulates SHP2 (e.g., a construct overexpressing SHP2) is provided. It inhibits the TLR4 inflammatory response by activating/up-regulating macrophage SHP2 and inhibiting TRAF6.

As a preferable embodiment of the present disclosure, based on the STING-TLR4 pathway in macrophages, a substance that activates/up-regulates STING (e.g., a construct overexpressing STING) is provided. It inhibits the TLR4 inflammatory response by activating/up-regulating macrophage STING.

As a preferable embodiment of the present disclosure, the sCD4 exerts its functions by regulating the MHCII/STING/SHP2 complex. Substances used to regulate the pathway can be used in the present disclosure as substances potentially useful for inhibiting macrophage hyperinflammatory responses. They can be chemical compounds, small chemical molecules, biomolecules. The biomolecules can be nucleic acids (including DNA, RNA) or proteins.

When used as targets for artificial regulation or for artificially establishing screening systems, the aforementioned proteins or encoding genes can be naturally occurring, for example, they can be purified and isolated from mammals; they can also be recombinantly prepared, for instance, recombinant proteins can be produced according to conventional gene recombination methods. Furthermore, any variants that do not affect the biological activity of these proteins are usable, such as derivatives or variants whose function remains unaltered.

### Drug Screening Based on Macrophage-Related Signaling Pathways

Based on the new discoveries of the present inventors, macrophage-related signaling pathway has been researched for multiple uses, wherein the uses comprise: screening for substances that regulate this signaling pathway, with the aim of using them to inhibit macrophage hyperinflammatory responses. Herein, the regulation comprises: up-regulating (e.g., activating or increasing expression of) MHC II, up-regulating SHP2, up-regulating STING, down-regulating (e.g., inhibiting or decreasing expression of) TRAF6, etc. The present disclosure provides a method for screening substances that inhibit inflammatory responses in macrophages, comprising: (1) contacting a candidate substance with a system comprising a signaling pathway selected from the group consisting of: MHC II-SHP2 and/or STING-TRAF6-TLR4 in macrophages; MHC II-TLR4 in macrophages; SHP2-TRAF6-TLR4 in macrophages; STING- TLR4 in macrophages; (2) observing the system in (1) and screening out substances that have a regulation on the signaling pathway in (1), wherein the substances (comprising potential substances) are useful for inhibiting inflammatory responses in macrophages. As used herein, terms "up-regulate", "down-regulate", "increase", "inhibit", "enhance", "weaken", "promote", "decrease" and such terms all refer to having statistical significance. That is: significantly "up-regulate", "down-regulate", "increase", "inhibit", "enhance", "weaken", "promote", "decrease". For example, compared to the protein activity, protein expression, protein binding or methylation level in a control group, significantly "up-regulate", "down-regulate", "increase", "inhibit", "enhance", "weaken", "promote", "decrease" by 10%, 20%, 30%, 40%, 50% or more; more preferably by 60%, 70%, 80%, 100% or more.

The system comprising the macrophage-related signaling pathway is selected from: a cellular system (or cell culture system), a subcellular system (or subcellular culture system), a solution system, an animal system or a tissue system (or tissue culture system).

As a preferable embodiment of the present disclosure, the method also comprises: further cell experiments and/or animal experiments on the obtained potential substances, so as to further select and determine substances really useful for inhibiting macrophage hyperinflammatory responses.

When performing screening, various methods well-known in the art can be used to determine changes in proteins or their encoding genes and their interactions.

Various conventional methods can be used to assess gene transcription or expression in the system. These methods comprise, but are not limited to: oligonucleotide hybridization methods (e.g., probes), polymerase chain reaction (PCR), polyacrylamide gel electrophoresis, etc. Detecting protein-protein interactions and the strength of these interactions can utilize various techniques well-known to those skilled in the art, such as co-immunoprecipitation, GST pull-down, phage display or the yeast two-hybrid system. Protein nuclear localization is also a method well-known in the art.

Substances preliminarily screened by the above methods can constitute a screening library, facilitating the eventual identification of substances that are truly useful for inhibiting macrophage hyperinflammatory responses.

The present disclosure also provides potential substances obtained using the screening method that can be used to inhibit macrophage hyperinflammatory responses.

The present disclosure further provides a method for preparing a drug for inhibiting macrophage hyperinflammatory responses (particularly inhibiting macrophage hyperinflammatory responses), wherein the method comprises: synthesizing and/or purifying the substance identified as useful for inhibiting macrophage hyperinflammatory responses by the aforementioned screening, to serve as a drug for inhibiting macrophage hyperinflammatory responses.

The obtained substance useful for inhibiting macrophage hyperinflammatory responses can be used to prepare a pharmaceutical composition, as described below in the present disclosure.

Methods for screening substances acting on a protein or gene or its specific region as a target are well known to those skilled in the art, and these methods can be used in the present disclosure. The candidate substances can be selected from: peptides, polymeric peptides, peptidomimetics, non-peptide compounds, carbohydrates, lipids, antibodies or antibody fragments, ligands, small organic molecules, small inorganic molecules, nucleic acid sequences, and so on. Based on the type of substances to be screened, it is clear to those skilled in the art how to select a suitable screening method.

The disclosure is further described below in conjunction with specific embodiments. It should be understood that these examples are merely for illustrative purposes rather than intended to limit the scope of the disclosure. The experimental methods without specifying the specific conditions in the following examples generally used the conventional conditions, such as those described in J. Sambrook, Molecular Cloning: A Laboratory Manual (3^{rd} ed. Science Press) or followed the manufacturer's recommendation.

### Materials and methods

### Pre-incubation of BMDM with sCD4 Protein

Differentiated BMDM were co-incubated overnight (8-12 hours) with 25 nM sCD4 protein in 24-well flat-bottom cell culture plates. Cells were then stimulated with 100 ng/mL LPS, 100 µg/mL poly(I:C), 0.3 µM CpG ODN or VSV (MOI=5) for 6-12 hours. Cell culture supernatants were collected for cytokine detection. BMDC cells were also used in specifically indicated experiments.

### Detection of TLR Pathway Modulation by sCD4 Protein via Western Blotting

Treated cells were lysed using cell lysis buffer to obtain total protein. Proteins were separated by 10% SDS-PAGE and transferred onto a PVDF membrane. The membrane was blocked with 5% skim milk. Primary antibody incubation was performed (generally using PBST with 5% skim milk for antibodies, or 5% BSA for phospho-specific antibodies) at 4°C with gentle shaking overnight. After incubation with corresponding secondary antibodies (in PBST with 5% skim milk) at room temperature for 1 hour, chemiluminescence was used for exposure and development.

### Administration of sCD4 Protein in Mice

sCD4 protein was administered via intraperitoneal injection at a dose of 15 µg per kg of mouse body weight, followed by intraperitoneal injection of LPS 12 hours later.

### Establishment of Mouse Sepsis Models

LPS Model: Eight-week-old male mice were used. LPS was administered intraperitoneally at a dose of 8 mg/kg body weight. Mortality was observed every 6 hours.

CLP Model: Eight-week-old male mice were anesthetized and fixed in a supine position on a surgical board. The abdominal surgical area was routinely disinfected and shaved. Under sterile conditions, a midline incision approximately 2 cm long was made in the abdominal wall. The cecum was exteriorized via the incision, and the distal one-third was ligated distal to the ileocecal valve using 3-0 silk suture. The ligated cecum was punctured once with an 18-gauge needle, and a small amount of fecal content was gently extruded. The peritoneum and skin were then sutured intermittently using 4-0 silk suture.

### Measurement of Inflammatory Cytokines by Luminex Assay

A standard sample was prepared first. The standard sample in a form of powder was centrifuged at 10,000 rpm for 10 seconds to pellet the protein. 200 µL of resuspension buffer was added, and the tube was vortexed thoroughly to mix. The solution was allowed to stand at room temperature for 10 minutes to ensure complete protein dissolution. The standard was then serially diluted in a 4-fold gradient to create 8 concentrations. Antibody-coated magnetic beads were vortexed thoroughly for 30 seconds to ensure homogeneity. 50 µL of the bead suspension was added to each well of a light-protected 96-well assay plate. The plate was placed on a magnetic stand for 2 minutes, and the supernatant was discarded. 150 µL of wash buffer was added to each well, incubated for 30 seconds, the plate was placed on the magnetic stand for 2 minutes, and the supernatant was discarded. 25 µL of standard or sample was added to each well. The plate was sealed with a plate sealer and incubated at 500 rpm at room temperature for 1 hour. The plate was placed on the magnetic stand for 2 minutes and the liquid was discarded. 150 µL of wash buffer was added to each well, incubated for 30 seconds, and the liquid was discarded. This wash step was repeated 3 times. 25 µL of detection antibody solution was added to each well, and the plate was incubated at 500 rpm at room temperature for 30 minutes. The plate was placed on the magnetic stand for 2 minutes, and the liquid was discarded. 150 µL of wash buffer was added, incubated for 30 seconds, the plate was placed on the magnetic stand for 2 minutes, and the supernatant was discarded. This wash step was repeated 3 times. 25 µL of Streptavidin-PE solution was added to each well, and the plate was incubated at 500 rpm at room temperature for 30 minutes. The plate was placed on the magnetic stand for 2 minutes, and the liquid was discarded. 150 µL of wash buffer was added, incubated for 30 seconds, and the liquid was discarded. This wash step was repeated 3 times. 120 µL of drive fluid was added to each well and the plate was analyzed using a Bio-Rad instrument.

### Sequence information

The amino sequence of murine sCD4 protein (SEQ ID NO: 1):

MCRAISLRRL LLLLLQLSQL LAVTQGKTLV LGKEGESAEL PCESSQKKIT VFTWKFSDQR
KILGQHGKGV LIRGGSPSQF DRFDSKKGAW EKGSFPLIIN KLKMEDSQTY ICELENRKEE
VELWVFKVTF SPGTSLLQGQ SLTLTLDSNS KVSNPLTECK HKKGKVVSGS KVLSMSNLRV
QDSDFWNCTV TLDQKKNWFG MTLSVLGFQS TAITAYKSEG ESAEFSFPLN FAEENGWGEL
MWKAEKDSFF QPWISFSIKN KEVSVQKSTK DLKLQLKETL PLTLKIPQVS LQFAGSGNLT
LTLDKGTLHQ EVNLVVMKVA QLNNTLTCEV MGPTSPKMRL TLKQENQEAR VSEEQKVVQV
VAPETGLWQC LLSEGDKVKM DSRIQVLSRG VNQT

The nucleotide sequence encoding murine sCD4 protein (SEQ ID NO: 2; codon-optimized sequence):

atgtgccgagccatctctcttaggcgcttgctgctgctgctgcagctgtcacaactcctagctgtcactcaagggaa
gacgctggtgctggggaaggaaggggaatcagcagaactgccctgcgagagttcccagaagaagatcacagtcttcacct
ggaagttctctgaccagaggaagattctggggcagcatggcaaaggtgtattaattagaggaggttcgccttcgcagttt
gatcgttttgattccaaaaaaggggcatgggagaaaggatcgtttcctctcatcatcaataaacttaagatggaagactc
tcagacttatatctgtgagctggagaacaggaaagaggaggtggagttgtgggtgttcaaagtgaccttcagtccgggta
ccagcctgttgcaagggcagagcctgaccctgaccttggatagcaactctaaggtctctaaccccttgacagagtgcaaa
cacaaaaagggtaaagttgtcagtggttccaaagttctctccatgtccaacctaagggttcaggacagcgacttctggaa
ctgcaccgtgaccctggaccagaaaaagaactggttcggcatgacactctcagtgctgggttttcagagcacagctatca
cggcctataagagtgagggagagtcagcggagttctccttcccactcaactttgcagaggaaaacgggtggggagagctg
atgtggaaggcagagaaggattctttcttccagccctggatctccttctccataaagaacaaagaggtgtccgtacaaaa
gtccaccaaagacctcaagctccagctgaaggaaacgctcccactcaccctcaagataccccaggtctcgcttcagtttg
ctggttctggcaacctgactctgactctggacaaagggacactgcatcaggaagtgaacctggtggtgatgaaagtggct
cagctcaacaatactttgacctgtgaggtgatgggacctacctctcccaagatgagactgaccctgaagcaggagaacca
ggaggccagggtctctgaggagcagaaagtagttcaagtggtggcccctgagacagggctgtggcagtgtctactgagtg
aaggtgataaggtcaagatggactccaggatccaggttttatccagaggggtgaaccagaca

The amino sequence of human sCD4 protein (SEQ ID NO: 3):

MNRGVPFRHL LLVLQLALLP AATQGKKVVL GKKGDTVELT CTASQKKSIQ FHWKNSNQIK
ILGNQGSFLT KGPSKLNDRA DSRRSLWDQG NFPLIIKNLK IEDSDTYICE VEDQKEEVQL
LVFGLTANSD THLLQGQSLT LTLESPPGSS PSVQCRSPRG KNIQGGKTLS VSQLELQDSG
TWTCTVLQNQ KKVEFKIDIV VLAFQKASSI VYKKEGEQVE FSFPLAFTVE KLTGSGELWW
QAERASSSKS WITFDLKNKE VSVKRVTQDP KLQMGKKLPL HLTLPQALPQ YAGSGNLTLA
LEAKTGKLHQ EVNLVVMRAT QLQKNLTCEV WGPTSPKLML SLKLENKEAK VSKREKAVWV
LNPEAGMWQC LLSDSGQVLL ESNIKVLPTW STPVQPMALI VLGGVAGLLL FI

The nucleotide sequence encoding human sCD4 protein (SEQ ID NO: 4; codon-optimized sequence):

ctctcttcatttaagcacgactctgcagaaggaacaaagcaccctccccactgggctcctggttgcagagctccaagtcc
tcacacagatacgcctgtttgagaagcagcgggcaagaaagacgcaagcccagaggccctgccatttctgtgggctcagg
tccctactggctcaggcccctgcctccctcggcaaggccacaatgaaccggggagtcccttttaggcacttgcttctggt

gctgcaactggcgctcctcccagcagccactcagggaaagaaagtggtgctgggcaaaaaaggggatacagtggaactga
cctgtacagcttcccagaagaagagcatacaattccactggaaaaactccaaccagataaagattctgggaaatcagggc
tccttcttaactaaaggtccatccaagctgaatgatcgcgctgactcaagaagaagcctttgggaccaaggaaactttcc
cctgatcatcaagaatcttaagatagaagactcagatacttacatctgtgaagtggaggaccagaaggaggaggtgcaat
tgctagtgttcggattgactgccaactctgacacccacctgcttcaggggcagagcctgaccctgaccttggagagcccc
cctggtagtagcccctcagtgcaatgtaggagtccaaggggtaaaaacatacagggggggaagaccctctccgtgtctca
gctggagctccaggatagtggcacctggacatgcactgtcttgcagaaccagaagaaggtggagttcaaaatagacatcg
tggtgctagctttccagaaggcctccagcatagtctataagaaagagggggaacaggtggagttctccttcccactcgcc
tttacagttgaaaagctgacgggcagtggcgagctgtggtggcaggcggagagggcttcctcctccaagtcttggatcac
ctttgacctgaagaacaaggaagtgtctgtaaaacgggttacccaggaccctaagctccagatgggcaagaagctcccgc
tccacctcaccctgccccaggccttgcctcagtatgctggctctggaaacctcaccctggcccttgaagcgaaaacagga
aagttgcatcaggaagtgaacctggtggtgatgagagccactcagctccagaaaaatttgacctgtgaggtgtggggacc
cacctcccctaagctgatgctgagtttgaaactgga

### Example 1. sCD4 inhibits TLR-mediated inflammatory responses in APCs (macrophages, dendritic cells).

### 1. sCD4 protein reduces inflammatory cytokine levels

The inventors co-cultured different concentrations of murine sCD4 protein (0, 25 nM, 50 nM, 250 nM) with mouse bone marrow-derived macrophages (BMDM, 5×10⁴ cells/well). After stimulating with 100 ng/mL LPS for 6 hours, the concentrations of inflammatory cytokines TNF and IL-6 in the cell supernatant were measured by Luminex assay.

As shown in Figure 1A, it can be seen that as the concentration of sCD4 protein increased, the production of inflammatory cytokines by the cells decreased significantly.

Therefore, sCD4 protein reduces inflammatory cytokine levels in a dose-dependent manner.

### 2. Inhibitory effect of sCD4 on inflammatory responses induced by different TLRs or in different cells

After co-culturing mouse BMDM with murine sCD4 protein, the inventors stimulated the cells with 100 µg/mL poly(I:C), 0.3 µM CpG or VSV virus (MOI = 5) to activate the TLR3, TLR7 and TLR9 signaling pathways, respectively, using a PBS group as control. The concentrations of inflammatory cytokines TNF and IL-6 in the cell supernatant were measured by Luminex assay.

As shown in Figure 1B, compared to the PBS control group, sCD4 protein significantly inhibited the intensity of the TLR3, TLR7, and TLR9 signaling pathways activated by poly(I:C), CpG or VSV virus. Among these, the inhibitory effect on IL-6 production induced by poly(I:C) or VSV virus was most pronounced.

Therefore, sCD4 protein inhibits the inflammatory responses caused by the TLR3, TLR7 and TLR9 signaling pathways activated by poly(I:C), CpG or VSV virus.

### 3. Inhibitory effect of sCD4 on inflammatory responses induced by different Toll-like receptors (TLRs) or in different cells

The inventors co-cultured different concentrations of murine sCD4 protein (0, 25 nM, 50 nM) with mouse bone marrow-derived dendritic cells (BMDC, 5×10⁴ cells/well), then challenged with LPS for the indicated duration. The concentrations of inflammatory cytokines TNF and IL-6 in the cell supernatant were measured by Luminex assay.

As shown in Figure 1C, sCD4 was detected to have a significant inhibitory effect on inflammatory responses triggered by different TLRs or in different cells.

### Example 2. Human sCD4 inhibits TLR4 inflammatory response in macrophages.

The inventors co-cultured different concentrations of human sCD4 protein (0, 1.39 nM, 4.17 nM, 12.5 nM, 37.5 nM, 112.5 nM) with human peripheral blood mononuclear cell (PBMC)-derived macrophages, then challenged with LPS for the indicated duration. The concentrations of inflammatory cytokines in the supernatant were measured by Luminex assay.

As shown in Figure 2, it can be observed that as the concentration of human sCD4 protein increased, the concentrations of TNF and IL-6 in the macrophage culture supernatant decreased very significantly. Therefore, human sCD4 inhibits the macrophage inflammatory response in a dose-dependent manner.

### Example 3. sCD4 protein effectively reduces mortality in LPS-induced sepsis.

The inventors intraperitoneally injected mice with murine sCD4 protein (15 µg/kg), followed by intraperitoneal injection of LPS 12 hours later. Mouse survival rates were observed at different time points. Concurrently, mouse serum was collected, and the concentrations of inflammatory cytokines TNF and IL-6 in the serum were analyzed at different time points.

The survival analysis results are shown in Figure 3A. The addition of sCD4 protein significantly improved animal survival rates.

The analysis results of TNF and IL-6 concentrations in serum are shown in Figure 3B. Significant decreases in TNF and IL-6 concentrations were observed at 12 and 24 hours after LPS injection, with the decrease at 24 hours being particularly pronounced.

### Example 4. sCD4 protein effectively reduces mortality in CLP-induced sepsis.

In this example, the effect of sCD4 protein was analyzed using a mouse sepsis model induced by Cecal Ligation and Puncture (CLP).

The inventors intraperitoneally injected mice with human sCD4 protein, then performed CLP surgery on the mice to establish the sepsis animal model. Serum and splenocytes were collected at the indicated time points. Inflammatory cytokine responses were detected by Luminex assay, and splenic macrophage activation was analyzed by flow cytometry.

The Luminex assay results are shown in Figure 4A. sCD4 significantly inhibited the inflammatory cytokine (TNF, IL-6) response in mice.

The flow cytometry analysis results are shown in Figure 4B. The number of resident macrophages in the sCD4 group did not change significantly, but the MHC II positive subset among them increased.

### Example 5: sCD4 Inhibition of TLR4 Inflammatory Response Depends on MHC II

The inventors co-cultured murine sCD4 protein (25 nM) with BMDM from either WT or MHC II knockout (MHC II^{-/-}) mice, followed by LPS challenge of the cells. As shown in Figure 5A, knockout of MHC II resulted in sCD4 being unable to prevent the TLR4 inflammatory response in MHCII^{-/-} BMDM cells. This indicates that sCD4 Inhibition of TLR4 Inflammatory Response Depends on MHC II

The inventors intraperitoneally injected sCD4 protein (15 µg/kg) into WT or MHC II^{-/-} mice, followed by LPS challenge. As shown in Figure 5B, after MHC II knockout, sCD4 protein failed to reduce mortality or the cytokine storm in MHCII^{-/-} mice.

Therefore, sCD4 inhibition of TLR4 inflammatory response depends on MHC II.

### Example 6. sCD4 inhibition of TLR4 inflammatory response depends on MHC II molecules in macrophages.

The inventors depleted macrophages in mice using clodronate liposomes, then adoptively transferred either MHC II^{+/+} or MHC II^{-/-} macrophages. Mice were intraperitoneally injected with murine sCD4 protein, followed by intraperitoneal LPS injection. Survival rates in mice and serum inflammatory cytokine levels were subsequently monitored.

As shown in Figures 6A-B, after adoptive transfer of MHC II^{+/+} macrophages, sCD4 significantly improved mouse survival rates and reduced inflammatory cytokine levels. In contrast, changes were not significant after transfer of MHC II^{-/-} macrophages.

Thus, sCD4 inhibition of TLR4 inflammatory response depends on MHC II molecules in macrophages.

### Example 7: sCD4 Inhibition of TLR4 Inflammatory Response Depends on CD40 signaling pathway.

The inventors co-incubated murine soluble CD40 ligand protein (sCD40L, obtained from Sino Biological) with BMDM from either WT or MHC II^{-/-} mice. After LPS challenge, inflammatory cytokine levels in the supernatant were detected by Luminex. As shown in Figure 7A, co-incubation with sCD40L significantly reduced inflammatory cytokine levels.

The inventors co-incubated sCD4 with BMDM from either WT or CD40 knockout (CD40^{-/-}) mice. After LPS challenge, the levels of inflammatory cytokines in the supernatant were detected by Luminex assay. As shown in Figure 7B, CD40 knockout (CD40^{-/-}) BMDM showed the same trend of cytokine changes as WT.

Therefore, sCD4 Inhibition of TLR4 inflammatory response depends on CD40 signaling pathway.

### Example 8. sCD4 modulates the TLR4 inflammatory response by activating SHP2 to inhibit TRAF6.

The inventors co-incubated wild-type BMDM with murine sCD4 protein (25 nM), then added LPS and challenged for different durations. The phosphorylation level of SHP2 in cell lysates was detected by Western blotting. As shown in Figure 8A, co-incubation with sCD4 protein promoted SHP2 phosphorylation, indicating that it can activate SHP2.

Furthermore, co-immunoprecipitation (co-IP) analysis for SHP2 and TRAF6 was performed to detect their interaction. As shown in Figure 8B, sCD4 protein significantly increased the interaction between SHP2 and TRAF6.

Furthermore, wild-type (SHP2^{fl/fl}) or macrophage-specific SHP2 knockout (SHP2^{-/-}) mice were intraperitoneally injected with sCD4 protein, followed by intraperitoneal LPS injection. Survival rate of mice and inflammatory response capacity were detected. As shown in Figure 8C, the ability of sCD4 to inhibit the TLR4 inflammatory response was lost upon macrophage-specific knockout of the SHP2 gene.

Furthermore, the phosphorylation levels of IRAK1 and IκBα were detected. As shown in Figure 8D, the ability of sCD4 to inhibit IRAK1 and IκBα phosphorylation was also lost upon macrophage-specific knockout of the SHP2 gene. Therefore, sCD4 controls the TLR4/IRAK1/NF-κB-mediated inflammatory response by activating SHP2 to inhibit TRAF6 activation.

### Example 9. sCD4 inhibition of TLR4 inflammatory response also depends on STING.

The inventors intraperitoneally injected murine sCD4 protein into WT (STING^{-/-}) or STING knockout (STING^{-/-}) mice, followed by intraperitoneal LPS injection. Survival rate of mice and inflammatory response capacity were detected.

As shown in Figure 9, administration of sCD4 in WT (STING^{-/-}) mice significantly improved animal survival rate, reaching 100% survival. In contrast, administration of sCD4 in the STING^{-/-} group showed no significant effect.

### Example 10. sCD4 disrupts the MHCII/TLR4 complex lipid raft domain and inhibits pro-inflammatory TLR4 plasma membrane localization.

BMDM were pre-incubated with sCD4 followed by LPS addition. As shown in Figure A, Duolink assay with antibodies against MHCII, TLR4, SHP2, phosphorylated SHP2 (pSHP2), STING, etc., followed by confocal imaging were used to quantitatively analyze pairwise interactions (red dots) between the indicated proteins in Figure A.Alternatively, as shown in Figure 10B, colocalization analysis (yellow) was performed in BMDM between Duolink-stained STING and SHP2 heterodimers (green) and immunofluorescently stained MHC II molecules (red).

The above results indicate that sCD4 modulates the MHCII/STING/SHP2 complex. sCD4 protein significantly inhibits the MHCII/TLR4 inflammatory complex, reduces the binding of the SHP2 and STING dimer to MHCII, but increases the binding of activated SHP2 (pY580) to MHCII. This suggests that sCD4 binding to MHCII disrupts the MHCII/TLR4 complex lipid raft domain and inhibits pro-inflammatory TLR4 plasma membrane localization.

### Example 11. sCD4 specifically interferes with TLR4 activation without affecting TNF inflammatory signaling.

The inventors co-incubated murine sCD4 protein with BMDM, then added TNF and stimulated for different durations. Changes in the inflammatory response in cell lysates, including phosphorylation levels of JNK, p38, and IκBα, were detected by Western blotting.

As shown in Figure 11, sCD4 specifically interferes with TLR4 activation without affecting TNF inflammatory signaling.

Above-described examples only show several embodiments of the present disclosure, which are described specifically and in detail. However, it should not be understood as a limiting patent scope of the present disclosure.

It should be noted that those skilled in the art can make some adjustments and improvements without departing from the concept of the present disclosure and all these forms are within the scope of protection of the present disclosure. Therefore, the scope of patent protection in the present disclosure should be determined by the appended claims. Simultaneously, each reference provided herein is incorporated by reference to the same extent as if each reference was individually incorporated by reference.

## Claims

1. A use of a recombinant soluble CD4 protein or a construct expressing the recombinant soluble CD4 protein in the manufacture of a pharmaceutical composition for preventing or treating inflammatory responses, wherein the inflammatory responses are septic hyperinflammation or inflammatory responses in macrophages.

2. The use according to claim 1, wherein the inflammatory responses in macrophages comprise: an inflammation caused by elevated expression of inflammatory factors mediated by TLR4 in macrophages in the onset of sepsis; preferably, the inflammation caused by elevated expression of inflammatory factors mediated by TLR4 in macrophages comprises: an inflammation caused by elevated TNF expression and/or an inflammation caused by elevated IL-6 expression.

3. The use according to claim 1, wherein the recombinant soluble CD4 protein comprises the ectodomain of the CD4 molecule; preferably, the ectodomain of the CD4 molecule comprises D1, D2, D3 and D4 domains; more preferably, the recombinant soluble CD4 protein is encoded by a gene codon-optimized coding sequence of human or mouse origin.

4. The use according to claim 1 or 3, wherein the recombinant soluble CD4 protein comprises a protein with the amino acid sequence shown in SEQ ID NO: 1 or SEQ ID NO: 3 or a conservative variant thereof; preferably, the conservative variant is selected from:
(1) a derivative protein formed by substituting, deleting or adding one or several amino acid residues in the amino acid sequence shown in SEQ ID NO: 1 or SEQ ID NO: 3 and retaining the function of the amino acid sequence shown in SEQ ID NO: 1 or SEQ ID NO: 3;
(2) a derivative protein with an amino acid sequence having more than 80% identity to the amino acid sequence shown in SEQ ID NO: 1 or SEQ ID NO: 3 and retaining the function of the amino acid sequence shown in SEQ ID NO: 1 or SEQ ID NO: 3; or
(3) a protein with a tag or a signal polypeptide fused to the protein with the amino acid sequence shown in SEQ ID NO: 1 or SEQ ID NO: 3.

5. The use according to claim 4, wherein the conservative variant is selected from: a variant comprising a small peptide tag that facilitates the detection of the protein with the amino acid sequence shown in SEQ ID NO: 1 or SEQ ID NO: 3, and/or an Fc-fused recombinant variant that enhances the stability of the protein with the amino acid sequence shown in SEQ ID NO: 1 or SEQ ID NO: 3.

6. The use according to claim 1, wherein the construct expressing soluble CD4 comprises an expression vector; preferably, the expression vector comprises a viral expression vector and a non-viral expression vector; more preferably, the viral expression vector comprises an adeno-associated viral vector, a lentiviral vector or an adenoviral vector.

7. The use according to claim 1, wherein the soluble CD4 inhibits the inflammatory response through crosstalk by binding to MHC II on macrophages; preferably, the inflammatory responses comprise septic hyperinflammation or inflammatory responses in macrophages; more preferably, the inflammatory responses in macrophages are TLR4-mediated inflammatory responses in macrophages.

8. The use according to claim 7, wherein the TLR4-mediated inflammatory responses are mediated by a signaling pathway of MHC II-TLR4 inflammatory response complex in macrophages; preferably, the signaling pathway comprises an up-regulation of MHC II by the recombinant soluble CD4 protein or the construct expressing soluble CD4, a recruitment and activation of SHP-2, an inhibition of TRAF6 and an inhibition of TLR4/NF-κB-mediated inflammatory responses through crosstalk.

9. A use of an artificially established or intracellular signaling pathway or a system comprising the signaling pathway for screening substances (comprising compounds, compositions, drugs, etc.) that inhibit inflammatory responses in macrophages; wherein the signaling pathway is selected from the group consisting of:
MHC II-SHP2 and/or STING-TRAF6-TLR4 in macrophages;
MHC II-TLR4 in macrophages;
SHP2-TRAF6-TLR4 in macrophages;
STING- TLR4 in macrophages.

10. A method for screening substances that inhibit inflammatory responses in macrophages, comprising:
(1) contacting a candidate substance with a system comprising a signaling pathway selected from the group consisting of:
MHC II-SHP2 and/or STING-TRAF6-TLR4 in macrophages;
MHC II-TLR4 in macrophages;
SHP2-TRAF6-TLR4 in macrophages;
STING- TLR4 in macrophages;
(2) observing the system in (1) and screening out substances that have a regulation on the signaling pathway in (1), wherein the substances (comprising potential substances) are useful for inhibiting inflammatory responses in macrophages; wherein, the regulation comprises up-regulation (such as activating or increasing expression) of MHC II, up-regulation of SHP2, up-regulation of STING, and down-regulation (such as inhibiting or decreasing expression) of TRAF6.
